# EUROPEAN PATENT SPECIFICATION

(11) **EP 3 509 544 B1**
(45) Date of publication and mention of the grant of the patent: **15.12.2021**
(21) Application number: 17847804.6
(22) Date of filing: 06.09.2017
(51) Int. Cl.: A61F 5/14, A43B 7/14, A43B 17/02

(54) **AN ORTHOTIC PRODUCT**
ORTHESEPRODUKT
PRODUIT ORTHÉTIQUE

(30) Priority: 06.09.2016 AU 2016903571
(43) Date of publication of application: 17.07.2019
(60) Divisional application: 21202926.8
(73) Proprietor: Kinetic Orthotics Pty Ltd, Marcoola, Queensland 4564 (AU)
(72) Inventor: EVERSON, Dan, Marcoola, Queensland 4564 (AU)
(74) Representative: Söltenfuss, Dirk Christian
(86) International application number: PCT/AU2017/000187
(87) International publication number: WO 2018/045411

(56) References cited:
- WO-A1-98/18358
- DE-C- 729 571
- DE-U1- 20 312 453
- US-A- 4 124 946
- US-A- 5 842 294
- US-A1- 2005 166 425
- US-A1- 2005 166 425
- US-A1- 2009 188 129
- US-A1- 2012 174 436
- US-A1- 2013 167 403
- US-A1- 2014 059 895
- US-A1- 2015 143 713
- US-A1- 2015 143 713
- US-A1- 2015 272 273
- US-A1- 2016 037 857
- US-B1- 6 286 232

## Description

### Field of the invention

The present invention relates to orthotics.

### Background of the invention

Orthotics are used in the treatment of foot disorders. Such orthotics are often provided as an insert that can be placed within the patient's shoes.

Many existing orthotics consider only the contour of the foot and the position of the foot when sitting or standing.

It is only recently that developments in orthotics have begun to consider other aspects of the foot, such as the position and movement of the foot in locomotion. For example, the applicant's own Australian Patent Application No. 2012262646 describes a process in which an individual is assessed using a number of functional tests. The results of those tests can then be used to fit the individual with an orthotic that can further improve the efficiency of the individual's gait cycle and therefore mobility.

Further, US 2015/0143713 A1 discloses a multi-function shoe pad composed of a main body for a user's human sole to tread, wherein the main body includes a hollow bulge integrally protruding toward a top side thereof and from a bottom side in such a way that a chamber is formed below the hollow bulge which allows the user's foot to tightly cling to the shoe to enable the shoe pad to be skidproof.

It is an object of this invention to provide an orthotic that has advantages over the prior art, or at least provides a useful alternative.

### Summary of the invention

This invention has come about due to the realisation of the inventor of the advantages of stimulating or blocking stimulation of certain parts of the foot, by means of targeted sections of an orthotic, to improve the movement pathway of an individual by improving the firing of the neuromuscular complex.

The subject-matter of the invention is defined by the appended independent claim, while some advantageous embodiments are defined in the dependent claims.

Further aspects of the claimed invention as well as further aspects being not part of the claimed invention will become apparent from the embodiments described in the detailed description and drawings.

A detailed description of one or more embodiments of the invention is provided below, along with accompanying figures that illustrate by way of example the principles of the invention.

For the purpose of example, numerous specific details are set forth in the following description in order to provide a thorough understanding of the present invention. The present invention may be practiced according to the claims without some or all of these specific details. For the purposes of clarity, technical material that is known in the technical fields related to the invention has not been described in detail so that the present invention is not unnecessarily obscured.

### Brief Description of Drawings

Various embodiments/aspects of the invention will now be described with reference to the following drawings in which:
Figure 1 is a bottom view of an orthotic in accordance with an embodiment of the present invention.
Figure 2 is a top view of an orthotic in accordance with an embodiment of the present invention.

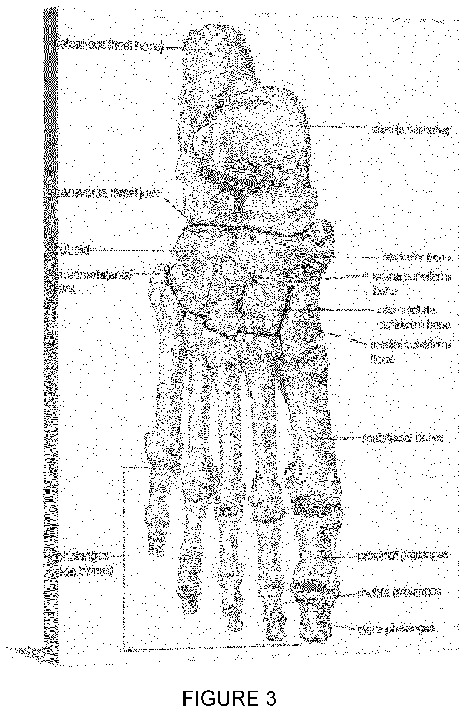

Figure 3 is a diagram of the bones of a foot.
Figures 4, 5, 7-9, 11-13 depict alternative embodiments of orthotics in accordance with the claimed invention, whereas Figures 6, 10, 14-19 depict alternative embodiments of orthotics being not part of the claimed.

### Detailed Description

The present invention will now be described with reference to one or more embodiments of the invention.

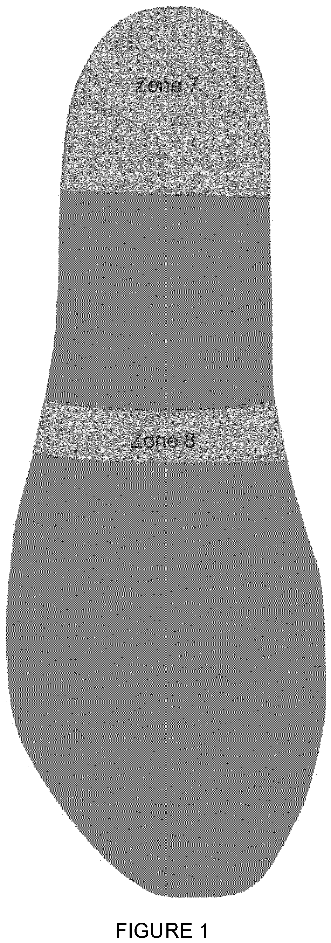

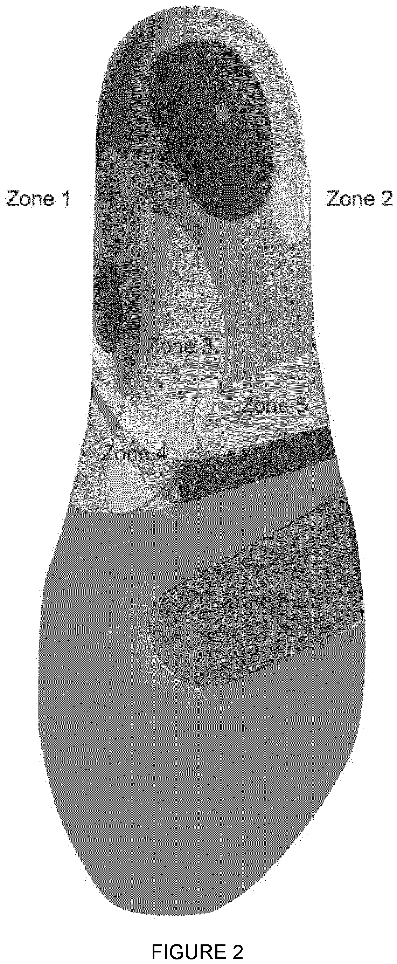

Figures 1 and 2 show the top side and bottom side of an orthotic in accordance with an embodiment of the present invention. The orthotic shows eight numbered zones, which correspond to particular features and locations on a patient's foot which have been identified as being important areas for neuro-stimulation. An orthotic in accordance with the present invention has a raised section or groove, as appropriate, in some of the identified zones.

The applicant has found that the stimulation or protection from stimulation, as appropriate, of particular locations of the foot corresponding to selected zone(s) improves the muscle function in order to achieve a more efficient gait cycle. Functional optimisation is achieved by improving the functional position of the foot. Firstly, by protecting and facilitating the windlass effect in conjunction with optimising the pivotal function at the key sagittal plane pivot sites in the foot; namely the heel, the ankle and the fore foot. Secondly, by stabilising the foot in the frontal plane in such a way as to create a functional equilibrium between the ability to efficiently pivot and the amount of support required (both in position and amplitude) to achieve this outcome.

A first zone is located in a position corresponding to the anterior of the medial side of the calcaneus and around the insertion site of the tibialis posterior into the side of the navicular of the foot. The zone is generally a substantially semicircular arc with a diameter that may vary from about 2 cm to about 4 cm. According to the present invention there is provided an orthotic with a raised section in this zone, which increases the support of the orthotic to facilitate the tibialis posterior muscle group. It should be noted that, for some feet, the raised section in this zone may be slightly longer (longitudinally) than shown in the figures.

A second zone is located in a position corresponding to the area of the foot around the cuboid area of the lateral side of the foot and extending anterior along one third of the shaft of the fifth metatarsal. In an embodiment of the present invention, a raised section is located in this zone. The stimulation of the foot by this raised zone works to stimulate the peroneal muscle insertions and tendons that track through this area. These insertions and tendons play an important role in stabilising the fore foot and the windlass effect during the propulsion phase of the gait cycle.

A third zone is a fascial groove zone starting in a location corresponding to the medial tubercle of the calcaneus to its insertion point into the base of the first metatarsal. The corresponding area on a foot is responsible for protecting the windlass effect. In an embodiment of the present invention, a groove is located in this zone. By having a groove in this zone, the pressure on the fascial chord of the foot can be minimised to reduce the incidence of uneven pressure on the chord interfering with the windlass mechanism.

A fourth functional zone is located at a position corresponding to the base of the first metatarsal zone and extending across approximately 1/3 of the width of the orthotic at the metatarsal area and one fifth of the length to the posterior heel point of the orthotic length on the medial side of the orthotic. According to the invention, there is an indentation in the orthotic in this zone. This decrease in bulk behind the first metatarsal resulting from the indentation encourages the plantar flexion of the first metatarsal.

The fifth zone is positioned at a location corresponding to 2 mm to 2 cm behind the second to fifth metatarsal heads on a foot. In an embodiment of the present invention, there is a raised surface in this zone. This raised surface increases the tension on the digital muscles in the foot as they insert into the metatarsal heads.

The sixth zone is located at a position corresponding to the sulci of a foot. In an embodiment of the present invention, there is a raised surface in this zone. The raised surface engages with the foot to enable better digital traction and reduce clawing of the toes. The raised section in the sixth zone may provide a wedging effect - for example, it may be higher on the inside or outside of the foot, to improve the forefoot position. Increasing the height of the raised section on the inside of the foot can be used to invert the forefoot position, or vice versa. This function of wedging the ball of the foot is quite different to the use of wedges in ordinary orthotics, where the is positioned behind the metatarsal. Note that, although (in the figures) the zone is shown as only extending about halfway laterally across the orthotic, for some people (with more severe forefoot position issues) the raised surface may be extended all the way across the orthotic.

The seventh zone is located on the bottom of the orthotic of the present invention in a position corresponding to the plantar heel surface of the foot. In an embodiment of the present invention, this zone is pitched from the rear of the orthotic to the front of the heel section. The pitched heel raise facilitates angle joint dorsiflexion.

The eighth zone is located on the bottom of the orthotic in a position corresponding to the location between the metatarsal phalangeal joints of the foot to 25 mm behind the phalangeal joints, and extends from the medial to the lateral size of the orthotic. In an embodiment of the present invention, an indentation extends across this entire zone. The presence of this indentation in the orthotic improves the rocker function in the forefoot to improve the efficiency of the gait of an individual and reduce the soft tissue stress on the foot. Although the feature has been described above as an indentation, in other embodiments it may simply be a flexion zone or hinge point, defined by a weakness or other arrangement (for example use of a more resilient material in the orthotic).

For each raised surface, the height of the raise is preferably greatest towards the centre of the respective zone - that is, there is a peak towards the centre of the zone. Each peak may be raised between 1 mm and 25 mm from the surrounding orthotic surface, but is preferably between 2 mm and 20 mm. and in most circumstances will be preferably between 3 mm and 8 mm.

Orthotics may be pre-made with the raised surfaces or grooves in all or most of the above-designed zones as it is anticipated that almost all patients in need of orthotics would benefit from stimulation/blocking of stimulation (as appropriate) in the identified zones.

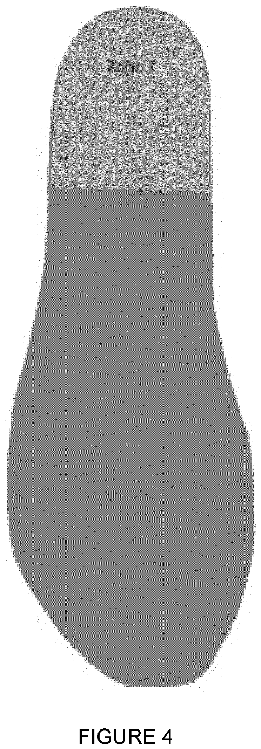

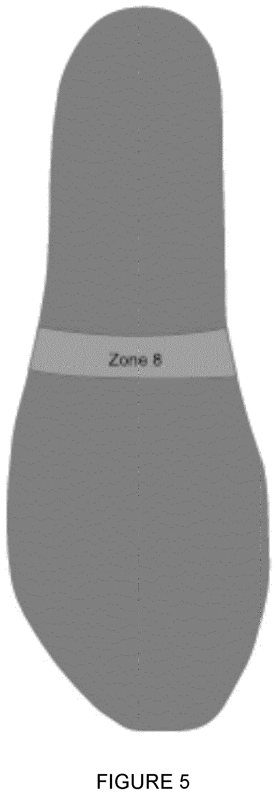

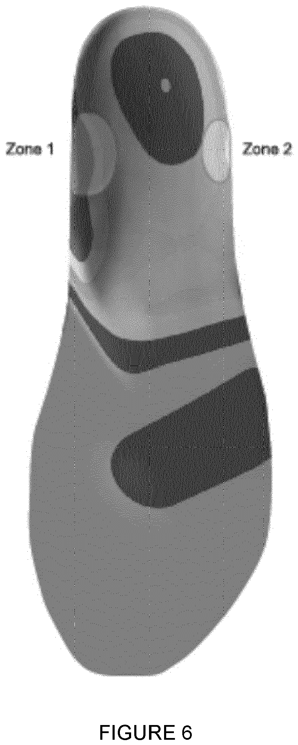

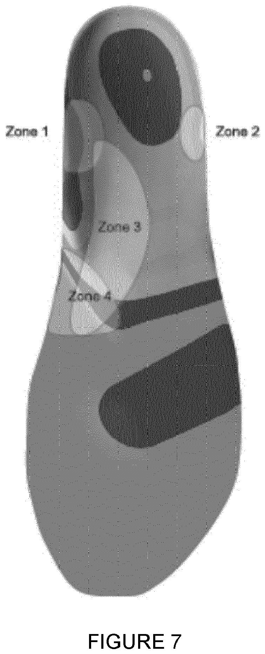

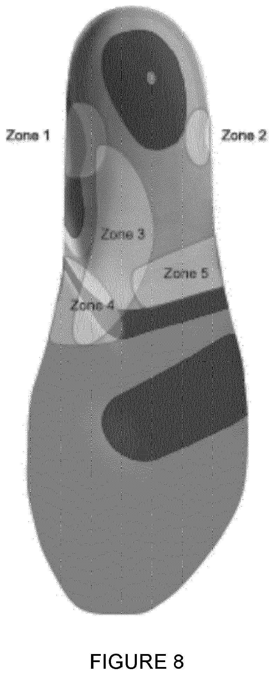

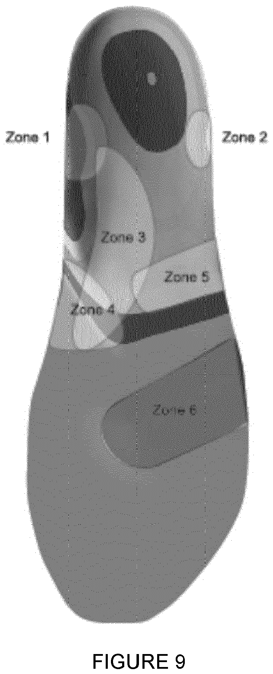

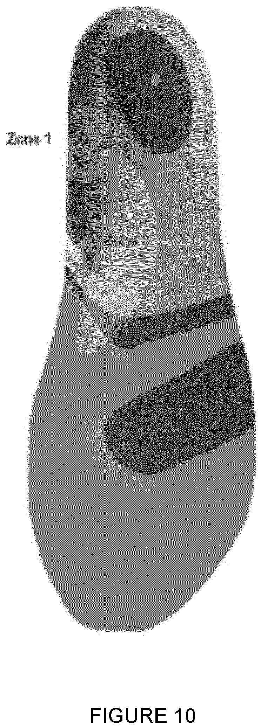

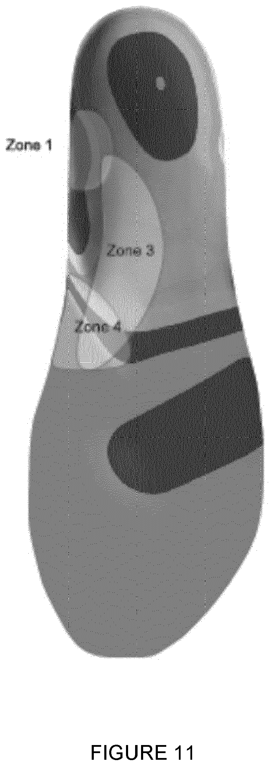

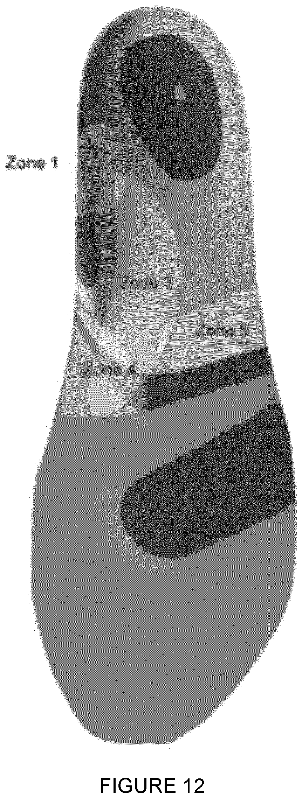

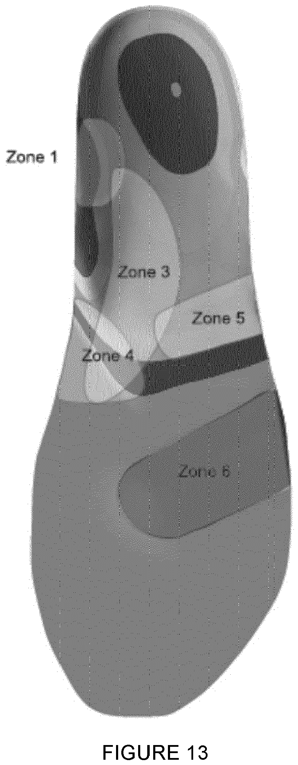

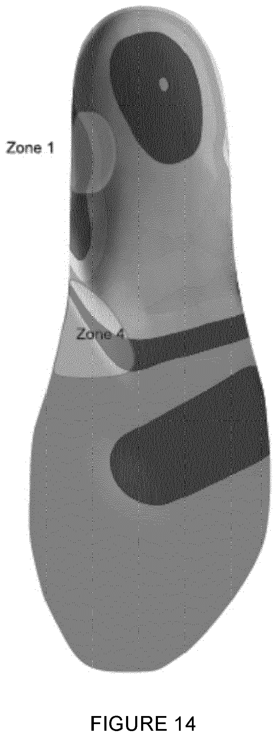

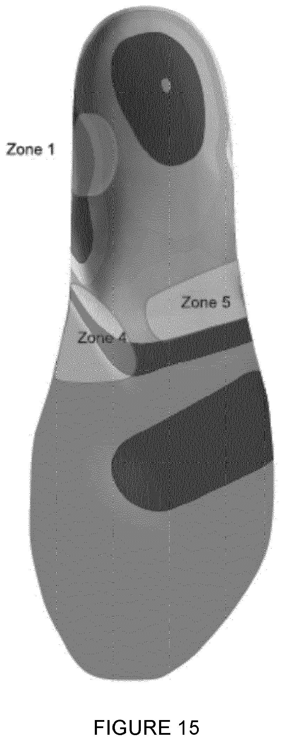

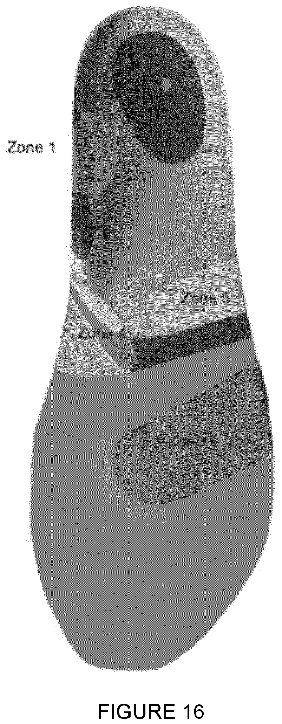

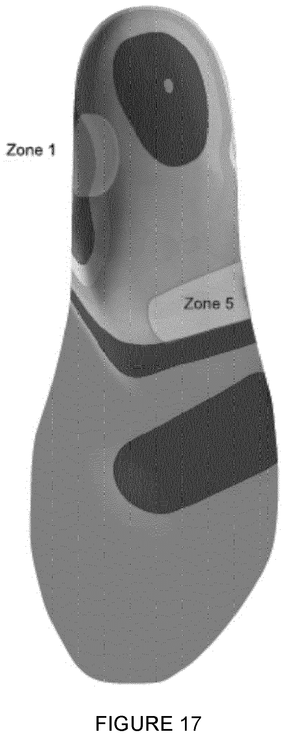

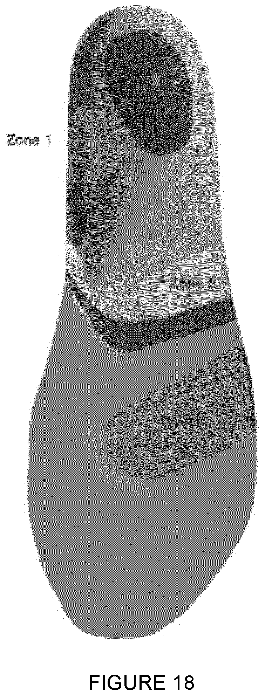

Alternatively, custom orthotics not belonging to the invention may be manufactured which include, amongst any other features suggested by the customised analysis, raised surfaces or grooves (as appropriate) in all or some of the above identified zones. For example, a patient may have a need for stimulation/blocking (as appropriate) in zones 7 only (Figure 4), zone 8 only (Figure 5), zones 1 and 2 only (Figure 6), zones 1 to 4 (Figure 7), zones 1 to 5 (Figure 8), zones 1 to 6 (Figure 9) zones 1 and 3 only (Figure 10), zones 1, 3 and 4 (Figure 11), zones 1, 3, 4 and 5 (Figure 12), zones 1, 3, 4, 5 and 6 (Figure 13), zones 1 and 4 only (Figure 14), zones 1, 4 and 5 (Figure 15), zones 1, 4, 5 and 6 (Figure 16), zones 1 and 5 only (Figure 17) or zones 1, 5 and 6 (Figure 18).

The orthotics shown in figures 4 to 18 are all full length orthotics. However, shorter orthotics (e.g. ¾ length, half-length, even ¼ length orthotics) may also be used in different embodiments of the invention, although shorter orthotics may not include all of the zones toward the front of the orthotic.

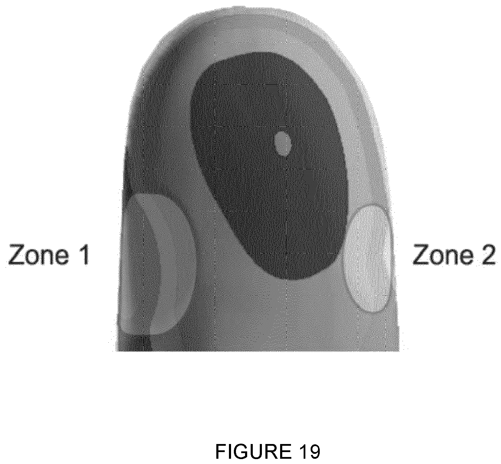

In Figure 19, there is depicted an orthotic having particular application in ballet, and other environments where small footwear worn such as in sprinting, cycling, or fashion. The orthotic in this embodiment not belonging to the invention is less than half length, and so takes up little room within the shoes and only has the first (calcaneal) and second (cuboid) "bumps" (i.e. raised sections). At least for a ballet application, no or minimal pitched heel may be used on the bottom of the orthotic. However, by stimulating of the foot using these bumps in zones 1 and 2, improved performance may be achieved in these applications.

The word 'comprising', and forms of the word 'comprising', when used in this specification is taken to specify the presence of stated features, integers, steps or components but does not preclude the presence or addition of one or more other features, integers, steps, components or groups thereof.

## Claims

1. An orthotic for a foot having a top side and a bottom side as well as a medial side and a lateral side, comprising:
a flexion zone (zone 8) in the bottom side of the orthotic located between the medial and the lateral side of the orthotic at a position corresponding to the location between a metatarsal phalangeal joint of the foot to 25mm behind the phalangeal joint, and/or a heel section (zone 7) in the bottom side of the orthotic which is pitched so that the height of the rear of the orthotic is greater than the height of the front of the heel section (zone 7); and further comprising a raised section in the top side of the orthotic located at a position corresponding to the cuboid area of the lateral side of the foot and extending in the anterior direction along one third of the shaft of the fifth metatarsal (zone 2), and/or a fascial groove located at a position corresponding to the area between the medial tubercle of the calcaneus to the insertion point of the medial cuneiform into the base of the first metatarsal (zone 3), wherein
the top side of the orthotic further has a raised section located at a position corresponding to the medial side of the calcaneus of the foot (zone 1), and an
indentation located at a position corresponding to the base of the first metatarsal of the foot (zone 4).

2. The orthotic according to claim 1, wherein the raised section in zone 1 is a substantially semicircular arc with a diameter of 2 to 4 cm.

3. The orthotic according to claim 1 or 2, further comprising a raised section located at a position corresponding to the sulci of the foot (zone 6).

4. The orthotic according to any one of claims 1 to 3, further comprising a raised section located at a position corresponding to 2 mm to 2 cm behind the second to fifth metatarsal heads of the foot (zone 5).

5. The orthotic according to any one of claims 1 to 4, wherein a height of the or each raised section in zone 1, 2, 5 and/or 6 is greatest towards the centre of the respective zone.

6. The orthotic according to claim 5, wherein the height of the or each raised section in zone 1, 2, 5 and/or 6 is less than 25 mm.

7. The orthotic according to claim 6, wherein the height of the or each raised section in zone 1, 2, 5 and/or 6 is between 2 mm and 20 mm.

8. The orthotic according to claim 7, wherein the height of the or each raised section in zone 1, 2, 5 and/or 6 is between 3 mm and 8 mm.

## Patentansprüche

1. Orthese für einen Fuß mit einer Oberseite und einer Unterseite sowie einer Innenseite und einer Außenseite, aufweisend:
eine Flexionszone (Zone 8) in der Unterseite der Orthese zwischen der Innen- und
der Außenseite der Orthese an einer Position entsprechend der Stelle zwischen einem Metatarsophalangealgelenk des Fußes bis 25 mm hinter dem Phalangealgelenk und/oder einen Fersenabschnitt (Zone 7) in der Unterseite der Orthese,
welcher so geneigt ist, dass die Höhe der Rückseite der Orthese größer ist als die Höhe der Vorderseite des Fersenabschnitts (Zone 7); und ferner aufweisend:
einen erhöhten Abschnitt in der Oberseite der Orthese an einer Position entsprechend dem Kuboidbereich der Außenseite des Fußes und sich in der Vorderrichtung entlang eines Drittels des Schafts des fünften Mittelfußknochens erstreckend (Zone 2) und/oder eine Fasziennut an einer Position entsprechend dem Bereich zwischen dem inneren Tuberkel des Fersenbeins zum Einfügepunkt des inneren Keilbeins in die Basis des ersten Mittelfußknochens (Zone 3),
wobei die Oberseite der Orthese ferner einen erhöhten Abschnitt an einer Position entsprechend der Innenseite des Fersenbeins des Fußes (Zone 1) und eine Vertiefung an einer Position entsprechend der Basis des ersten Mittelfußknochens des Fußes (Zone 4) hat.

2. Orthese nach Anspruch 1, bei welcher der erhöhte Abschnitt in Zone 1 ein im Wesentlichen halbkreisförmiger Bogen mit einem Durchmesser von 2 bis 4 cm ist.

3. Orthese nach Anspruch 1 oder 2, ferner aufweisend einen erhöhten Abschnitt an einer Position entsprechend den Furchen des Fußes (Zone 6).

4. Orthese nach einem der Ansprüche 1 bis 3, ferner aufweisend einen erhöhten Abschnitt an einer Position entsprechend 2 mm bis 2 cm hinter den zweiten bis fünften Mittelfußknochenköpfen des Fußes (Zone 5).

5. Orthese nach einem der Ansprüche 1 bis 4, bei welcher eine Höhe des oder jedes erhöhten Abschnitts in Zone 1, 2, 5 und/oder 6 zur Mitte der jeweiligen Zone am größten ist.

6. Orthese nach Anspruch 5, bei welcher die Höhe des oder jedes erhöhten Abschnitts in Zone 1, 2, 5 und/oder 6 weniger als 25 mm beträgt.

7. Orthese nach Anspruch 6, bei welcher die Höhe des oder jedes erhöhten Abschnitts in Zone 1, 2, 5 und/oder 6 zwischen 2 mm und 20 mm beträgt.

8. Orthese nach Anspruch 7, bei welcher die Höhe des oder jedes erhöhten Abschnitts in Zone 1, 2, 5 und/oder 6 zwischen 3 mm und 8 mm beträgt.

## Revendications

1. Orthèse pour un pied ayant un côté supérieur et un côté inférieur ainsi qu'un côté médial et un côté latéral, laquelle comprend :
une zone de flexion (zone 8) qui est sur le côté inférieur de l'orthèse et située entre le côté médial et le côté latéral de l'orthèse à une position correspondant à l'emplacement entre une articulation métatarsienne phalangienne du pied et 25 mm en arrière de l'articulation phalangienne, et / ou une section de talon (zone 7) qui est sur le côté inférieur de l'orthèse et inclinée de sorte que la hauteur de l'arrière de l'orthèse est supérieure à la hauteur de la face avant de la section de talon (zone 7), et comprend en outre :
une section surélevée qui est sur le côté supérieur de l'orthèse et située à une position correspondant à la zone cuboïde du côté latéral du pied et s'étendant dans la direction antérieure le long d'un tiers du corps du cinquième métatarsien (zone 2), et / ou une rainure fasciale qui est située à une position correspondant à l'aire entre le tubercule médial du calcanéum et le point d'insertion du cunéiforme médial dans la base du premier métatarsien (zone 3),
dans laquelle :
le côté supérieur de l'orthèse a en outre une section surélevée qui est située à une position correspondant au côté médial du calcanéum du pied (zone 1), et une indentation qui est située à une position correspondant à la base du premier métatarsien du pied (zone 4).

2. Orthèse selon la revendication 1, dans laquelle la section surélevée dans la zone 1 est sensiblement un arc semi-circulaire d'un diamètre de 2 à 4 cm.

3. Orthèse selon la revendication 1 ou 2, comprenant en outre une section surélevée qui est située à une position correspondant aux sillons du pied (zone 6).

4. Orthèse selon l'une quelconque des revendications 1 à 3, comprenant en outre une section surélevée qui est située à une position correspondante allant de 2 mm à 2 cm derrière la deuxième jusqu'à la cinquième tête métatarsienne du pied (zone 5).

5. Orthèse selon l'une quelconque des revendications 1 à 4, dans laquelle une hauteur de la section surélevée dans les zones 1, 2, 5 et / ou 6 ou une hauteur de chaque section surélevée dans les zones 1, 2, 5 et / ou 6 est la plus grande en allant vers le centre de la zone respective.

6. Orthèse selon la revendication 5, dans laquelle la hauteur de la section surélevée dans les zones 1, 2, 5 et / ou 6 ou de chaque section surélevée dans les zones 1, 2, 5 et / ou 6 est inférieure à 25 mm.

7. Orthèse selon la revendication 6, dans laquelle la hauteur de la section surélevée dans les zones 1, 2, 5 et / ou 6 ou de chaque section surélevée dans les zones 1, 2, 5 et / ou 6 est comprise entre 2 mm et 20 mm.

8. Orthèse selon la revendication 7, dans laquelle la hauteur de la section surélevée dans les zones 1, 2, 5 et / ou 6 ou de chaque section surélevée dans les zones 1, 2, 5 et / ou 6 est comprise entre 3 mm et 8 mm.
